# EUROPÄISCHE PATENTSCHRIFT

(11) **EP 1 589 022 B1**
(45) Veröffentlichungstag und Bekanntmachung des Hinweises auf die Patenterteilung: **25.02.2015**
(21) Anmeldenummer: 05005427.9
(22) Anmeldetag: 12.03.2005
(51) Int. Cl.: C07H 15/04, A61K 31/60

(54) **Verfahren zur Herstellung eines Gemischs enthaltend Fettalkohole und APG- ethercarboxylat**
Process for the production of a mixture comprising fatty acid alcohols and APG-ether-carboxylate
Procédé de préparation d'un mélange comprenant d'alcools gras et d' éthers carboxyliques d'alkylpolyglycosides

(30) Priorität: 23.03.2004 DE 102004014013
(43) Veröffentlichungstag der Anmeldung: 26.10.2005
(73) Patentinhaber: Cognis IP Management GmbH, 40589 Düsseldorf (DE)
(72) Erfinder: Behler, Ansgar, 46240 Bottrop (DE); Schmid, Karl Heinz, 40822 Mettmann (DE); Neuss, Michael, 50997 Köln (DE); Eskuchen, Rainer, 40764 Langenfeld (DE)

(56) Entgegenhaltungen:
- WO-A-02/090369
- WO-A-03/043725
- Product Data Sheet PLANTACARE 1200 UP

## Beschreibung

Die vorliegende Erfindung betrifft ein Verfahren zur Herstellung eines Gemisches enthaltend einen oder mehrere verschiedene Fettalkohole und APG-ethercarboxylat.

APG-ethercarboxylate sind aus Stand der Technik bekannte Tenside. APG-ethercarboxylat ist die Abkürzung für Alkylpolyglycosid-ethercarboxylat oder Alkenylpolyglycosidethercarboxylat. APG-ethercarboxylate sind dadurch erhältlich, dass APG (APG steht für Alkylpolyglycosid oder Alkenylpolyglycosid) an einer oder mehreren OH-Gruppen des Polyglycosidrestes des APG derivatisiert werden, indem diese eine oder mehrere OH-Gruppen mit je einem Molekül ω-Halogencarbonsäure oder deren Salz oder deren Ester zu einem Ether umgesetzt werden und die Carbonsäuregruppe der ω-Halogencarbonsäure in eine Carboxylatgruppe überführt wird.

Das APG, aus dem APG-ethercarboxylate auf die geschilderte Weise hergestellt werden können, nennt man "das dem APG-ethercarboxylat zu Grunde liegende APG". Die Substituenten des APG-ethercarboxylates, die sich von der ω-Halogencarbonsäure ableiten, nennt man "Ethercarboxylgruppen".

Die Zahl der Ethercarboxylgruppen die ein Molekül APG-ethercarboxylat trägt, ist in einer bestimmten Probe von APG-ethercarboxylat verschieden und ist durch die Herstellung bestimmt. In einer bestimmten Probe APG-ethercarboxylat mit einem bestimmten statistischen Mittel an Ethercarboxylgruppen je ein Molekül APG-ethercarboxylat schwankt die Zahl der Ethercarboxylgruppen pro Molekül APG-ethercarboxylat von Molekül zu Molekül.

APG-ethercarboxylate sind üblicherweise keine Reinstoffe, weil in ein und derselben Substanzprobe üblicherweise verschiedene Fettalkoholreste, verschiedene Kettenlängen der Polyglycosidreste und verschieden hohe Substitutionsgrade mit Ethercarboxylat-Gruppen vorliegen. Wenn im Singular von APG-ethercarboxylat gesprochen wird, dann bedeutet das nicht, dass ein Reinstoff vorliegt.

APG können durch folgende Formel (I) beschrieben werden:

R¹O-[G]ₚ (I),

in der R¹ für einen Alkylrest oder für einen Alkenylrest mit bevorzugt 4 bis 22 Kohlenstoffatomen, G für einen Zuckerrest mit bevorzugt 5 oder 6 Kohlenstoffatomen und p für eine Zahl von bevorzugt 1 bis 10 steht. APG können nach üblichen Verfahren hergestellt werden. Die Herstellung von APG ist zum Beispiel beschrieben in der Publikation von Biermann et al. in Starch/Stärke 45, 281 (1993), und in der Publikation von B. Salka in Cosm. Toil. 108, 89 (1993) und in der Publikation von J. Kahre et al. in SÖFW-Journal Heft 8, 598 (1995).

Bei der Herstellung von APG wird im allgemeinen der Fettalkohol im Überschuss eingesetzt und nach der Reaktion durch Destillation aus dem Produkt vollständig entfernt bzw. sein Gehalt im Produktgemisch wird durch Destillation reduziert. Das "Roh-APG" enthält nach der Reaktion, vor der Destillation, üblicherweise noch ca. 50 bis 80 % Fettalkohol.

Die Herstellung von APG-ethercarboxylat kann durch Umsetzung von APG mit ω-Halogencarbonsäuren im alkalischen Medium erfolgen. Da APG im geschmolzenen Zustand sehr hohe Viskositäten aufweisen, werden üblicherweise bei der Umsetzung geeignete Lösungsmittel zugesetzt. Im allgemeinen werden organische, aprotische Lösungsmittel verwendet, so dass eine Hydrolyse der Halogencarbonsäuren bzw. deren Salze oder Ester vermieden wird. In WO 97/42299 wird die Umsetzung mit Toluol als Lösungsmittel beschrieben.

WO 02/090369 offenbart ein Verfahren zur Herstellung von APG-ethercarboxylat durch die Umsetzung von APG mit einer ω-Halogencarbonsäure, deren Salz oder Ester in wässriger Lösung.

WO 03/043725 offenbart Gemische aus APG und Fettalkoholen und deren Verwendung.

Der vorliegenden Erfindung liegt die Aufgabe zu Grunde ein neues Verfahren zur Herstellung von APG-ethercarboxylat bereitzustellen.

Diese Aufgabe wird gelöst durch ein Verfahren gemäß Anspruch 1.

Dabei sind die Gegenionen zu den APG-ethercarboxylat-Anionen bevorzugt AlkalimetallKationen sind, besonders bevorzugt Natrium-Kationen.

Dieses Verfahren ist ein Gegenstand der vorliegenden Erfindung. Es wird erfindungsgemäßes Verfahren genannt.

Eine Ausführungsform der Erfindung ist das erfindungsgemäße Verfahren, wobei der Anteil des APG-ethercarboxylates an dem enthaltenen Gemisch 10 bis 90 Gew.-%, bevorzugt 20 bis 80 Gew.-%, besonders bevorzugt 20 bis 60 Gew.-%, beträgt.

Eine weitere Ausführungsform der Erfindung ist das erfindungsgemäße Verfahren, wobei der Anteil der Fettalkohole an dem enthaltenen Gemisch 20 bis 80 Gew.-%, besonders bevorzugt 40 bis 80 Gew.-%, beträgt.

Eine weitere Ausführungsform der Erfindung ist das erfindungsgemäße Verfahren, wobei das dem APG-ethercarboxylat zu Grunde liegende APG die Formel (I) hat

R¹O-[G]ₚ (I),

in der R¹ für einen Alkylrest oder für einen Alkenylrest mit 4 bis 22 Kohlenstoffatomen, bevorzugt mit 8 bis 18 C-Atomen (bevorzugt ist R¹ ein Gemisch aus Hexadecylresten und Octadecylresten), steht, G für einen Zuckerrest mit 5 oder 6 Kohlenstoffatomen (bevorzugt sind Zuckerreste G abgeleitet von Aldosen oder Ketosen mit 5 bis 6 C-Atomen, besonders bevorzugt ist G abgeleitet von Glukose) und p für eine Zahl von 1 bis 10 (bevorzugt 1,1 bis 3) steht.

Die Indexzahl p in Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden APG mit einem mittleren Oligomerisierungsgrad p von 1,1 bis 3,0 eingesetzt. Aus anwendungstechnischer Sicht sind solche APG bevorzugt, deren Oligomerisierungsgrad kleiner als 1,7 ist und insbesondere zwischen 1,2 und 1,4 liegt.

Eine weitere Ausführungsform der Erfindung ist das erfindungsgemäße Verfahren, wobei das enthaltene Gemisch als Fettalkohole eine Mischung aus C 8- bis C 20-Fettalkohole, bevorzugt C 14- bis C 20-Fettalkohole, enthält.

Eine Ausführungsform der Erfindung ist das erfindungsgemäße Verfahren, wobei die Umsetzung in Gegenwart einer Base (bevorzugt Natriumhydroxid oder Kaliumhydroxid, besonders bevorzugt Natriumhydroxid) erfolgt.

Eine weitere Ausführungsform der Erfindung ist das erfindungsgemäße Verfahren, wobei die ω-Halogencarbonsäure, das Salz einer ω-Halogencarbonsäure oder der Ester einer ω-Halogencarbonsäure eine Verbindung mit 2 bis 10, bevorzugt 2 bis 6, besonders bevorzugt 2 C-Atomen ist (Natriummonochloracetat ist besonders bevorzugt).

Eine weitere Ausführungsform der Erfindung ist das erfindungsgemäße Verfahren, wobei der Anteil von d (also Fettalkohole) bezogen auf die Summe aus c und d 40 bis 80 Gew.-%, beträgt.

Eine weitere Ausführungsform der Erfindung ist das erfindungsgemäße Verfahren, wobei das Molverhältnis von der Verbindung ausgewählt aus der Gruppe bestehend aus einer ω-Halogencarbonsäure, dem Salz einer ω-Halogencarbonsäure und dem Ester einer ω-Halogencarbonsäure und dem dem APG-ethercarboxylat zu Grunde liegende APG 0,5 zu 1 bis 3,5 zu 1, bevorzugt 1 zu 1 bis 2,5 zu 1 ist.

Eine weitere Ausführungsform der Erfindung ist das erfindungsgemäße Verfahren, wobei die Umsetzung bei 50 bis 130 °C, bevorzugt bei 80 bis 110 °C erfolgt.

Eine weitere Ausführungsform der Erfindung ist das erfindungsgemäße Verfahren, wobei die Umsetzung ohne Lösungsmittel (außer den vorhandenen Fettalkoholen d) erfolgt.

Ein weiterer Gegenstand der vorliegenden Erfindung ist ein Verfahren zur Herstellung von APG-ethercarboxylat umfassend
a) das erfindungsgemäße Verfahren und
b) die Entfernung der Fettalkohole aus dem Gemisch,
wobei die Entfernung der Fettalkohole aus dem Gemisch bevorzugt durch Destillation erfolgt.

Überraschenderweise wurde gefunden, dass die Umsetzung von APG mit einer Verbindung ausgewählt aus der Gruppe bestehend aus einer ω-Halogencarbonsäure, dem Salz einer ω-Halogencarbonsäure und dem Ester einer ω-Halogencarbonsäure auch ohne Zusatz von Wasser oder sonstigen Lösungsmitteln möglich ist, wenn das APG als Gemisch enthaltend das APG und einen oder mehrere verschiedenen Fettalkohole eingesetzt wird. Bevorzugt wird dabei das sogenannte "Roh-APG" eingesetzt, das bei der Herstellung des APG vor der destillativen Entfernung des Fettalkohols anfällt, und das noch bevorzugt 50 bis 80 Gew.-% Fettalkohol enthält. Dabei hat es sich gezeigt, dass nahezu ausschließlich das APG mit der Verbindung ausgewählt aus der Gruppe bestehend aus einer ω-Halogencarbonsäure, dem Salz einer ω-Halogencarbonsäure und dem Ester einer ω-Halogencarbonsäure zu APG-ethercarboxylat reagiert und der Fettalkohol nahezu oder ganz unverändert aus der Reaktion hervorgeht.

Es ist ein Vorteil des erfindungsgemäßen Verfahrens zur Herstellung von APG-ethercarboxylat, dass das Roh-APG aus der APG-Herstellung eingesetzt werden kann, ohne das zuvor der Fettalkohol entfernt werden muss. Außerdem werden keine Lösungsmittel benötigt.

Das nach dem erfindungsgemäßen Verfahren erhältliche Gemisch kann als Tensid in oberflächenaktiven Zubereitungen, wie beispielsweise Wasch- und Spülmittel, Haushaltsreiniger sowie kosmetische und/oder pharmazeutische Zubereitungen eingesetzt werden. Diese oberflächenaktiven Zubereitungen können als weitere Hilfs- und Zusatzstoffe Perlglanzwachse, Konsistenzgeber, Verdickungsmittel, Überfettungsmittel, Stabilisatoren, Siliconverbindungen, Fette, Wachse, Lecithine, Phospholipide, Antioxidantien, Deodorantien, Antitranspirantien, Antischuppenmittel, Quellmittel, Tyrosininhibitoren, Hydrotrope, Solubilisatoren, Konservierungsmittel, Parfümöle, Farbstoffe, weitere Tenside und dergleichen enthalten. Als kosmetische und/oder pharmazeutische Zubereitungen kommen beispielsweise Mund- und Zahnpflegemittel, Haarshampoos, Haarlotionen, Schaumbäder, Duschbäder, Cremes, Gele, Lotionen, alkoholische und wässrig/alkoholische Lösungen und Emulsionen in Frage.

Gemische enthaltend APG-ethercarboxylat und einen oder mehrere verschiedene Fettalkohole eignen sich für die Anwendungen, die in WO 03/043725 für die Gemische gemäß WO 03/043725 offenbart sind. Insbesondere sind dies Anwendungen in kosmetischen oder pharmazeutischen Formulierungen.

### Beispiele

### %-Angaben in den Beispielen sind Gew.-%

Für die Beispiele wurde C16/18-APG verwendet. Das ist ein APG, das durch die Umsetzung von Glukose mit C14/20-Fettalkohol erhalten wird. C14/20-Fettalkohol ist ein Gemisch verschiedener Fettalkohole mit folgender Zusammensetzung:

| | |
|---|---|
| C-14-Fettalkohol | max. 3 % |
| C-16-Fettalkohol | 45 - 55 % |
| C-18-Fettalkohol | 45 - 55 % |
| C-20-Fettalkohol | max. 3 % |

Der Restgehalt an Fettalkohol im C16/18-APG betrug 59,5 %.

### Beispiel 1: Herstellung von C16/18-APG-ethercarboxylat

In einem Reaktionsgefäß wurden 749,1 g C16/18-APG mit Restgehalt an Fettalkohol (wie oben) von 78 % (0,33 mol) und 22,4 g Natrium-hydroxid-Microprills (0,56 mol) auf eine Temperatur von 85 °C erwärmt. Anschließend wurde unter Rühren portionsweise 65,3 g Natriumchloracetat (0,56 mol) über einen Zeitraum von 4 Stunden zugegeben. Nach 3 Stunden Nachreaktionszeit und mit Erreichen der theoretischen Mengen an freigesetztem Chlorid (2,32 % Cl) war die Reaktion abgeschlossen.

Folgende analytische Daten wurden bestimmt:
- Fettalkohol-Gehalt im APG-ethercarboxylat: 64,5 %
- Gehalt an NaCl: 3,6 %
- Nicht umgesetztes APG im Produktgemisch: 5,50 % (das entspricht einem Umsatz von 72,1 %)

### Beispiel 2: Herstellung von C16/18-APG-ethercarboxylat

In einem Reaktionsgefäß wurden 500,0 g C16/18-APG mit einem Fettalholgehalt (wie oben) von 64,1 % (0,37 mol) und 19,1 g Natriumhydroxid-Microprills (0,48 mol) auf eine Temperatur von 115 °C erwärmt. Anschließend wurde unter Rühren 55,7 g Natriumchloracetat (0,48 mol) zugegeben. Nach 4 Stunden Nachreaktionszeit und mit Erreichen der theoretischen Mengen an freigesetztem Chlorid (2,94 % Cl) war die Reaktion abgeschlossen.

Folgende analytische Daten wurden bestimmt:
- Fettalkohol-Gehalt im APG-ethercarboxylat: 53,0 %
- Gehalt an NaCl: 4,8 %
- Nicht umgesetztes APG im Produktgemisch: 6,6 % (das entspricht einem Umsatz von 78,8 %)

### Beispiel 3: Herstellung von C16/18-APG-ethercarboxylat

In einem Reaktionsgefäß wurden 493,4 g C16/18-APG (0,4 mol) mit einem Fettalkoholgehalt von 59,5 % und 27,2 g Natriumhydroxid-Microprills (0,68 mol) auf eine Temperatur von 100 °C erwärmt. Anschließend wurde unter Rühren portionsweise 79,2 g Natriumchloracetat (0,68 mol), über einen Zeitraum von 4 Stunden zugegeben. Nach 3 Stunden Nachreaktionszeit und mit Erreichen der theoretischen Mengen an freigesetztem Chlorid (3,95 % Cl) war die Reaktion abgeschlossen.

Folgende analytische Daten wurden bestimmt:
- Fettalkohol-Gehalt im APG-ethercarboxylat: 52,2%
- Gehalt an NaCl: 6,5 %
- Nicht umgesetztes APG im Produktgemisch: 5,0 % (das entspricht einem Umsatz von 85 %)

## Patentansprüche

1. Ein Verfahren zur Herstellung eines Gemisches enthaltend
a) APG-ethercarboxylat und
b) einen oder mehrere verschiedene Fettalkohole.
umfassend die Umsetzung eines Ausgangsgemisches enthaltend
c) das dem APG-ethercarboxylat zu Grunde liegende APG (APG steht für Alkylpolyglycosid oder Alkenylpolyglycosid) und
d) einen oder mehrere verschiedenen Fettalkohole
mit einer Verbindung ausgewählt aus der Gruppe bestehend aus einer ω-Halogencarbonsäure, dem Salz einer ω-Halogencarbonsäure und dem Ester einer ω-Halogencarbonsäure, wobei der Anteil von d (also Fettalkohole) bezogen auf die Summe aus c und d 10 bis 80 Gew.-% beträgt.

2. Das Verfahren nach Anspruch 1, wobei das dem APG-ethercarboxylat zu Grunde liegende APG die Formel (I) hat
R¹O-[G]ₚ (I),
in der
R¹ für einen Alkylrest oder für einen Alkenylrest mit 4 bis 22 Kohlenstoffatomen steht,
G für einen Zuckerrest mit 5 oder 6 Kohlenstoffatomen und
p für eine Zahl von 1 bis 10 steht.

3. Das Verfahren nach Anspruch 1 oder 2, wobei die Umsetzung in Gegenwart einer Base erfolgt.

4. Das Verfahren nach einem der Ansprüche 1 bis 3, wobei die ω-Halogencarbonsäure, das Salz einer ω-Halogencarbonsäure oder der Ester einer ω-Halogencarbonsäure eine Verbindung mit 2 bis 10 C-Atomen ist.

5. Das Verfahren nach einem der Ansprüche 1 bis 4, wobei das Molverhältnis von der Verbindung ausgewählt aus der Gruppe bestehend aus einer ω-Halogencarbonsäure, dem Salz einer ω-Halogencarbonsäure und dem Ester einer ω-Halogencarbonsäure
und dem dem APG-ethercarboxylat zu Grunde liegende APG 0,5 zu 1 bis 3,5 zu 1 ist.

6. Das Verfahren nach einem der Ansprüche 1 bis 5, wobei die Umsetzung ohne Lösungsmittel (außer den vorhandenen Fettalkoholen d) erfolgt.

7. Ein Verfahren zur Herstellung von APG-ethercarboxylat umfassend
a) das Verfahren nach einem der Ansprüche 1 bis 6 und
b) die Entfernung der Fettalkohole aus dem Gemisch.

## Claims

1. A process for producing a mixture comprising
a) APG ether carboxylate and
b) one or more different fatty alcohols, comprising the reaction of a starting mixture comprising
c) the APG on which the APG ether carboxylate is based (APG stands for alkyl polyglycoside or alkenyl polyglycoside) and
d) one or more different fatty alcohols with a compound selected from the group consisting of an ω-halocarboxylic acid, the salt of an ω-halocarboxylic acid and the ester of an ω-halocarboxylic acid, where the fraction of d (i.e. fatty alcohols), based on the sum of c and d, is 10 to 80% by weight.

2. The process according to claim 1, where the APG on which the APG ether carboxylate is based has the formula (I)
R¹O-[G]ₚ, (I),
in which
R¹ is an alkyl radical or an alkenyl radical having 4 to 22 carbon atoms,
G is a sugar radical having 5 or 6 carbon atoms and
p is a number from 1 to 10.

3. The process according to claim 1 or 2, where the reaction takes place in the presence of a base.

4. The process according to any one of claims 1 to 3, where the ω-halocarboxylic acid, the salt of an ω-halocarboxylic acid or the ester of an ω-halocarboxylic acid is a compound with 2 to 10 carbon atoms.

5. The process according to any one of claims 1 to 4, where the molar ratio of the compound selected from the group consisting of an ω-halocarboxylic acid, the salt of an ω-halocarboxylic acid and the ester of an ω-halocarboxylic acid
and the APG on which the APG ether carboxylate is based
is 0.5:1 to 3.5:1.

6. The process according to any one of claims 1 to 5, where the reaction takes place without solvents (apart from the fatty alcohols d present).

7. A process for producing APG ether carboxylate, comprising
a) the process according to any one of claims 1 to 6 and
b) the removal of the fatty alcohols from the mixture.

## Revendications

1. Procédé de fabrication d'un mélange contenant :
a) un éther-carboxylate d'APG et
b) un ou plusieurs alcools gras différents, comprenant la mise en réaction d'un mélange de départ contenant :
c) l'APG à la base de l'éther-carboxylate d'APG (APG signifie alkylpolyglycoside ou alcénylpolyglycoside) et
d) un ou plusieurs alcools gras différents,
avec un composé choisi dans le groupe constitué par un acide ω-halogénocarboxylique, le sel d'un acide ω-halogénocarboxylique et l'ester d'un acide ω-halogénocarboxylique, la proportion de d (c'est-à-dire d'alcools gras) par rapport à la somme de c et d étant de 10 à 80 % en poids.

2. Procédé selon la revendication 1, dans lequel l'APG à la base de l'éther-carboxylate d'APG a la formule (I)
R¹O- [G]ₚ (I)
dans laquelle
R¹ représente un radical alkyle ou un radical alcényle de 4 à 22 atomes de carbone,
G représente un radical sucre de 5 ou 6 atomes de carbone et
p représente un nombre de 1 à 10.

3. Procédé selon la revendication 1 ou 2, dans lequel la réaction a lieu en présence d'une base.

4. Procédé selon l'une quelconque des revendications 1 à 3, dans lequel l'acide ω-halogénocarboxylique, le sel d'un acide ω-halogénocarboxylique ou l'ester d'un acide ω-halogénocarboxylique est un composé de 2 à 10 atomes C.

5. Procédé selon l'une quelconque des revendications 1 à 4, dans lequel le rapport molaire entre le composé choisi dans le groupe constitué par un acide ω-halogénocarboxylique, le sel d'un acide ω-halogénocarboxylique et l'ester d'un acide ω-halogénocarboxylique et l'APG à la base de l'éther-carboxylate d'APG est de 0,5 sur 1 à 3,5 sur 1.

6. Procédé selon l'une quelconque des revendications 1 à 5, dans lequel la réaction a lieu sans solvant (à l'exception des alcools gras d présents).

7. Procédé de fabrication d'un éther-carboxylate d'APG comprenant :
a) le procédé selon l'une quelconque des revendications 1 à 6 et
b) l'élimination des alcools gras du mélange.
